Europäisches Patentamt

(19) European Patent Office

Office européen des brevets

(11) Publication number: 0 325 086
A2

# (12) EUROPEAN PATENT APPLICATION

(21) Application number: 88810741.4

(22) Date of filing: 28.10.88

(51) Int. Cl.⁴ A61K 9/22 , A61K 9/32 , A61K 9/36

(30) Priority: 23.11.87 CH 4554/87

(43) Date of publication of application:
26.07.89 Bulletin 89/30

(84) Designated Contracting States:
AT BE CH DE ES FR GB GR IT LI LU NL SE

(71) Applicant: JAGO RESEARCH AG
Seestrasse 47
CH-6052 Hergiswil(CH)

(72) Inventor: Gonella, Jacques, Dr.
Bahnhofstrasse 29
CH-8702 Zollikon(CH)

(74) Representative: Frei, Alexandra Sarah
Frei Patentanwaltsbüro Hedwigsteig 6
Postfach 95
CH-8029 Zürich(CH)

(54) Novel methods for obtaining therapeutic systems with controlled release of the drug.

(57) Therapeutic system for the controlled rate release of drugs, characterized by:

1. a polymeric matrix consisting of hydrophilic, swelling, natural and/or synthetic, biocompatible polymers containing the therapeutically active dose of drug,

2. a polymeric barrier applied on said matrix and bound to decompose when in contact with aqueous fluids which controls the flow of the fluids into the interior of the system only at the beginning of the release period.

EP 0 325 086 A2

### "Novel methods for obtaining therapeutic systems with controlled release of the drug"

A novel method is described for obtaining a controlled rate release, for a period of time determinable in advance, of an active ingredient on an appropriate carrier.

Such a novel system is characterized by:

1. a polymeric matrix consisting of hydrophilic, swelling, natural and/or synthetic, biocompatible polymers containing the therapeutically active dose of drug,

2. a polymeric barrier applied on said matrix and bound to decompose when in contact with aqueous fluids which controls the flow of the fluids into the interior of the system only at the beginning of the release period.

The active ingredient in said system is released almost entirely according to nearly zero-order kinetics.

Moreover, the process for preparing said system is described in detail. In the pharmaceutical field the possibility of a controlled rate administration of an active ingredient is very important, because, with such a system, the therapeutic effect of a drug can be optimized by maintaining effective hematic levels of the same after oral administration.

The matrix-type systems, with a drug release kinetics approximating the zero-order, are convenient for a controlled release.

For obtaining a drug release which is statistically nearer to the zero-order, pharmaceutical forms are produced composed by entities carrying the dose of drug, their outer surface being modified in order to alter the inherent kinetic of the matrix.

In fact, one main characteristic of the matrix release is the initial rate of active ingredient delivery which is very high compared with the subsequent release time; such a trend is mainly responsible for the release deviating from a linear kinetics.

Attempts can be made in order to reach said kinetics by employing devices of suitable geometry, hydrophilic porous materials or hydrophilic, scarcely soluble polymers, which all are able to vary the actual diffusion of the active ingredient.

Compression-prepared matrices, their outer surface being modified by a cross-linking reaction caused by exposure to UV radiation or by thermic treatment, are described in detail as minimatrices for the controlled administration of a drug in Journal of Controlled Release 1, 4, 283(1985).

The object of the present invention is to prepare matrices carrying known active ingredients by employing known polymeric materials and to alter the outer surface of the matrix by application of biodegradable films in order to obtain a nearly zero-order kinetics for the release of the ingredient in the matrix. To this end, a polymeric barrier has been applied on the matrix, according to known production procedures. Said barrier, the permeability of which changes during the operation of the system, represents a structural obstacle to the diffusion of the drug in the initial release period, but, owing to its subsequent degradation and/or decomposition, it does not hamper any more the drug delivery in the following release period.

We have, in fact, tried to overcome all the disadvantages of the "reservoir" systems, which tipically release the active ingredient according to a zero-order kinetics for a portion of their content, but present a rather long "leg time" and a drug residue not suitable for administration at constant release.

Object of the present invention is a novel therapeutic system for the controlled release of drugs.

Said systems consists of:

1. a matrix structure obtained by compressing a mixture of the active ingredient and appropriate polymeric materials (matrix or core),

2. a polymeric coating applied on the above defined structure (matrix or core), which forms a degradable and/or soluble barrier for controlling the drug release only in the initial period of the matrix release.

the polimeric material employed for the core preparation shall belong to the class of pharmaceutical, swelling, biocompatible and/or biodegradable polymers and is selected from the group of methylcelluloses of various molecular weights, polyvinylalcohols, acrylic copolymers, hydroxypropylmethylcelluloses and generally any type of natural or synthetic polymers, including ethylcellulose, cellulose acetate-phtalate, which can form a matrix by compression.

In addition to the active ingredient and the polymeric material other components can be employed in the formulations, which are usually classified as technological aids for an easier processing of the mixture (compressibility, etc.).

Furthermore, in order to make easier the flow of water and/or gastric and/or enteric fluids into the structure of the matrix, it can be necessary, expecially in the case of not very soluble drugs, the addition of compatible, water-soluble additives, which can form, on dissolution, a structure of pores and canals aiding in the release of the active ingredient and in the hydration of the polymeric material. The polymeric coating of the matrix has the purpose of forming a temporary barrier.

Such a barrier can have different composition and structure according to its operative life. Strongly hydrophilic and easily permeable membranes will allow a fast flow of water and aqueous fluids into the interior, consequently making easier a fast release of the active ingredient, and will be preferred with not very soluble active ingredients. Membranes composed by hydrophobic or poorly hydrophilic, less water-permeable, polymeric materials will be used with soluble or very soluble active ingredients.

The polymeric materials which can be employed for the membrance include hydroxypropylmethylcellulose, hydroxypropylcellulose, methylcellulose, acrylic polymers and copolymers of various molecular weights, ethylcellulose, cellulose acetate-phtalate.

The operation of the system according to the invention can be outlined as follows: the active ingredient release begins upon immersion in aqueous fluids and is controlled in this first period by the membrane which forms a barrier applied on the core. The water permeating through said barrier brings about concomitantly the drug release and the progressive weakening of the barrier. when the strenght of the membrane reaches its limit, its consequent degradation and/or solubilization allows the freeing of the core which carries on the release of the drug, not more controlled by the membrane, but by the structure of the matrix forming the core.

As a consequence, the drug release can take place gradually without any high initial rates, the carried portion of the drug being completely released without the insoluble residues of the "reservoir" systems.

The global release rate can therefore be controlled by both the core and membrance compositions.

The invention is described in detail in the following indicative, but not limitative example. The example refers only to a known drug, but obviously the present invention can be applied to a great number of other active ingredients to be employed either in the medical or other fields (agricultural, etc....).

## Example 1

For preparing a lot of 100 novel, pharmaceutical, controlled release forms the following, quali-quantitative formulation has been employed:

| Active ingredient | |
|---|---|
| - Diltiazem | 180 g |
| Excipients | |
| - Hydroxypropylmethylcellulose | 200 g |
| - mannitol | 10 g |
| - Magnesium stearate | 4 g |
| - Methylcellulose | 5 g |
| - Ethanol/water (1:1) | 140 g |

## Matrix preparation

The active ingredient is mixed in a suitable mixing apparatus with the polymeric material and mannitol previously sieved through a 300 μm sieve. The resulting powdered mixture is wetted with 200 g of ethanol/water (1:1) and the obtained homogeneous mass is forced through a screen of 425 μm. The resulting granules, dried in an oven at 30-35° C, are added with 0,5% of magnesium stearate lubricant.

The mixture is pressed according to known technique using 11 mm diameter, flat punches at a compressive force of 2000 kg/cm$^2$.

## Matrix coating

3

For coating the matrix it has been employed a suspension of the following composion:

| | |
|---|---|
| - acrilic copolymer soluble at pH 5-7 (Eudragit -Rohm Pharma) | 6 g |
| - Castor oil | 0,5 g |
| - FDC 4 lacquer | 0,2 g |
| - acetone - isopropanol 1:1 balance to 100 ml | |

The coating is carried out in a coating pan according to the usual technological procedures till systems are obtained having a smooth, continuous, coating membrane, about 50 $\mu$m thick.

Release control

The release control is carried out by employing the apparatus 11 of the United States Pharmacopea USP XXI (paddle), with distilled water at 37° C and a rotational speed of 100 rpm. The released active ingredient is determined by spectrophotometry with continuous-flow cells.

The release test are carried out on:
a. matrices without controlling barrier
b. matrices with controlling barrier

| Release from not-coated matrices | |
|---|---|
| Time (min) | Released portion (cumulative %) |
| 15 | 28,8 |
| 30 | 46,2 |
| 60 | 72,3 |
| 90 | 89,6 |
| 120 | 100,0 |

| Release from barrier-coated matrices | |
|---|---|
| Time (min) | Released portion (cumulative %) |
| 30 | 20,0 |
| 60 | 40,9 |
| 90 | 57,1 |
| 120 | 69,5 |
| 180 | 98,0 |

## Claims

1. A therapeutic system for the controlled rate release of drugs consisting of:
a. a matrix reservoir containing, in addition to the active ingredient, suitable polymeric materials able to provide by compression a heterogeneous matrix,
b. a polymeric coating applied on the core surface and forming a barrier for the temporary control of the drug release.

2. A therapeutic system according to claim 1, characterized in that the polymeric material of a., forming the reservoir core which produces the matrix structure by compression, is selected from the group of the high-viscosity hydroxypropylmethylcellulos, methylcelluloses, polyvinylalcohols, acrylic copolimers, hydroxypropylmethylcelluloses, etc.

3. A therapeutic system according to claim 1, characterized in that the polymeric material of 5., which forms the membrane, includes acrylic polymers and copolymers, ethylcellulose, methylcellulose, hydroxypropylmethylcellulose, hydroxypropylcellulose, cellulose acetate-phtalate.

4. A therapeutic system according to claims 1 and 3, characterized in that the application of the polymeric coating b. on the reservoir-core is carried out by atomization of a polymeric solution in a coating pan according to known technique.

5. A therapeutic system according to claims 1, 3 and 4, characterized in that the application of polymeric coating b. on the reservoir-core is carried out according to the fluidized bed technique or other known techniques.

6. A therapeutic system according to claim 1, characterized in that the reservoir-core is obtained by compression.

7. A therapeutic system according to claims 1-6, characterized in that said coated matrices lose their coating during teh release period following a chemical or mechanical degradation.

8. A therapeutic system according to claim 1-7, characterized in that the matrix continues to control the drug release according to a diffusive mechanism, when the system has lost its coating.